# EUROPÄISCHE PATENTSCHRIFT

(11) **EP 3 170 518 B1**
(45) Veröffentlichungstag und Bekanntmachung des Hinweises auf die Patenterteilung: **28.07.2021**
(21) Anmeldenummer: 16197524.8
(22) Anmeldetag: 07.11.2016
(51) Int. Cl.: A61M 1/16

(54) **DIALYSEMASCHINE MIT INTEGRIERTER DIALYSATORKUPPLUNG**
DIALYSIS MACHINE WITH INTEGRATED DIALYSIS MACHINE COUPLING
DIALYSEUR COMPRENANT UN DISPOSITIF D'ACCOUPLEMENT INTÉGRÉ DE DIALYSEUR

(30) Priorität: 23.11.2015 DE 102015120218
(43) Veröffentlichungstag der Anmeldung: 24.05.2017
(73) Patentinhaber: B. Braun Avitum AG, 34212 Melsungen (DE)
(72) Erfinder: STENZEL, Bruno, 34346 Hann. Münden (DE); BRÖGGER, Sebastian, 34593 Knüllwald (DE); LUDWIG, Uta, 37287 Wehretal (DE)
(74) Vertreter: Winter, Brandl - Partnerschaft mbB

(56) Entgegenhaltungen:
- EP-A1- 0 887 100
- WO-A2-01/08722
- WO-A2-2009/039357
- DE-A1- 3 642 671
- US-A1- 2014 112 828
- US-A1- 2014 217 029

## Beschreibung

Die Erfindung betrifft eine extrakorporale Blutbehandlungsmaschine, insbesondere Dialysemaschine mit einer maschineninternen Fluidik für eine Blutbehandlungsflüssigkeit, vorzugsweise Dialysierflüssigkeit und einem die Fluidik zumindest bereichsweise oder abschnittsweise kapselnden Gehäuse (Maschinengehäuse), wobei die Fluidik eine Zuleitung für frische Blutbehandlungsflüssigkeit (nachfolgend grundsätzlich als Frischdialysierflüssigkeit bezeichnet) zu einem Filter/Dialysator und eine Ableitung für verbrauchte Blutbehandlungsflüssigkeit (nachfolgend grundsätzlich als Brauchdialysierflüssigkeit bezeichnet) vom Filter/Dialysator aufweist, die jeweils endseitig mit einem Anschluss zur lösbaren Kopplung des Filters/Dialysators versehen sind.

Bei heutigen Dialyseanwendungen werden Dialysemaschinen genutzt, bei denen ein Kartuschen-artiger Filter/Dialysator zunächst mit einer Klemme vorzugsweise außenseitig am Dialysegerät bzw. am Gehäuse der Dialysemaschine befestigt wird. Der so an der Dialysemaschine befestigte Dialysator wird anschließend strömungstechnisch mit der Internfluidik der Dialysemaschine verbunden, indem Schläuche mit Schnellverbindern einerseits mit dafür bestimmten Anschlüssen des Dialysators und andererseits mit dafür bestimmten Anschlüssen der Internfluidik der Dialysemaschine verbunden werden, welche sich ebenfalls am Gehäuse der Dialysemaschine befinden. Diese Dialysierflüssigkeit führenden Schläuche (nachfolgend auch als maschinenseitige Schläuche bezeichnet) liegen neben Blutschläuchen (nachfolgend auch als patientenseitige Schläuche bezeichnet) im Nahbereich des Dialysators vor. Da die Schläuche in der täglichen Praxis in der Regel eher wirr angeordnet sind und/oder sich leicht verwirren können, ist keine besonders hohe Benutzerfreundlichkeit gegeben. Ein weiterer Nachteil bei einer Verwendung von Dialysierflüssigkeitsschläuchen ist, dass es durch die Länge der Schläuche und deren schlechte thermische Isolationseigenschaften in der Regel zu Temperaturverlusten der Dialysierflüssigkeit kommen kann, was dann wieder zu einer Temperaturverringerung des in den patientenseitigen Schläuchen geführten Bluts und damit zu einem Auskühlen des Patienten führen kann. Ein anderer Nachteil ist, dass es in der täglichen Praxis zu Störungen zum Beispiel durch ein Abklemmen oder Abreißen von Schläuchen an Betten, Tischen oder sonstigen Störkonturen kommen kann.

Zudem ist es bei bekannten Lösungen möglich, dass ungeeignete Filtermodule für die jeweilige Dialysemaschine eingesetzt werden.

Die Druckschrift US 2014/0112828 A1 beschreibt eine Dialyseeinheit mit einem Gehäuse, welches eine Dialysatorhalterung in Form von schlüssellochähnlichen Führungen aufweist, in welche an einem Dialysator angebrachte Schnellverschlusskupplungen eingehängt werden können, wobei der Dialysator zu diesem Zeitpunkt jedoch bereits angeschlossen sein muss und somit ein Hantieren mit entsprechenden Schläuchen nicht vermeidbar ist.

Die US 2014/217029 A1 zeigt einen Fluidkreislauf für eine modular aufgebaute Dialyseeinheit, mit im Gehäuse integrierten Anschlüssen, wobei eine Kassette, welche unter anderem auch den Filter enthält, gegen ein Reinigungselement ausgetauscht werden kann.

Weiterer Stand der Technik findet sich in den Veröffentlichungen WO 2009/039357 A2, WO 01/08722 A2, EP 0 887 100 A1 und DE 36 42 671 A1.

Ausgehend von dem vorstehend beschriebenen Stand der Technik liegt der vorliegenden Erfindung die Aufgabe zugrunde, die zuvor angeführten Nachteile zu beseitigen, insbesondere eine extrakorporale Blutbehandlungsmaschine, vorzugsweise Dialysemaschine bereit zu stellen, mittels der eine Verbesserung der Anwenderfreundlichkeit bewirkt wird, die besonders übersichtlich ist und eine nutzerseitige Einhandbedienung ermöglicht. Des Weiteren sollen Temperaturverluste der maschinenseitigen Blutbehandlungsflüssigkeit/Dialysierflüssigkeit im Zu-/Ablauf des Filters, d.h. im Dialysatorzu- und -ablauf verringert oder gar vermieden werden.

Diese Aufgabe wird prinzipiell durch eine extrakorporale Blutbehandlungsmaschine mit den Merkmalen des Patentanspruchs 1 gelöst. Vorteilhafte Weiterbildungen der Erfindung sind Gegenstand der Unteransprüche.

Insbesondere wird diese Aufgabe gelöst durch eine Blutbehandlungsmaschine/ Dialysemaschine mit einer maschineninternen Fluidik für Blutbehandlungsflüssigkeit/Dialysierflüssigkeit und einem die Fluidik zumindest bereichsweise oder abschnittsweise kapselnden/einschließenden Gehäuse (Maschinengehäuse), wobei die Fluidik eine Zuleitung für Frischdialysierflüssigkeit zu einem Filter/Dialysator und eine Ableitung für Brauchdialysierflüssigkeit vom Filter/Dialysator aufweist, die jeweils endseitig mit einem Anschluss zur lösbaren Kopplung des Filters/Dialysators versehen sind. Erfindungsgemäß ist dieser Anschluss der Zuleitung und/oder dieser Anschluss der Ableitung jeweils als in das Gehäuse (Maschinengehäuse) integrierte Anschluss-Kupplung ausgebildet. D.h. die Anschlüsse der Blutbehandlungsmaschine/Dialysemaschine für den Filter/Dialysator befinden sich erfindungsgemäß nicht mehr an den freien Enden maschinenexterner Verbindungschläuche, sondern sitzen (unmittelbar) auf/in der Außenseite einer Gehäusewand des die Fluidik einschließenden Maschinengehäuses, derart, dass der Filter/Dialysator ohne Zwischenfügen von externen Verbindungsschläuchen (direkt) an die Anschlüsse der Blutbehandlungsmaschine fluidtechnisch und ggf. auch mechanisch angekoppelt werden kann.

Des Weiteren sind die maschinenseitigen Anschluss-Kupplungen in einer außenseitig im Gehäuse ausgebildeten oder angeordneten konkaven Vertiefung / Mulde / Ausnehmung angeordnet, welche eine Art Aufnahmeschale für den (Kartuschen-artigen) Filter/Dialysator bildet. Die Tiefe einer solchen Vertiefung ist (vorzugsweise leicht/geringfügig) tiefer als die Höhe der Anschluss-Kupplung(en) in dieser Richtung ausgehend vom Vertiefungsgrund, so dass die Anschluss-Kupplung(en) vollständig in die Vertiefung versenkt und geschützt ist/sind. Die Vertiefung ist derart hinsichtlich ihrer Größe und Geometrie ausgebildet, dass sie die Aufnahmeschale/-mulde für den Dialysator ausbildet, deren Dimensionierung/Abmessung an den für die Blutbehandlungsmaschine konkret vorgesehenen Filter/Dialysator angepasst ist. Dadurch kann die Verwendung eines ungeeigneten Filters/Dialysators mit entsprechend unpassenden Abmessungen verhindert werden.

Vereinfacht kann man sagen, dass die Grundidee, auf der die Erfindung beruht, darin besteht, die Dialysierflüssigkeitsanschlüsse des vorzugsweise Kartuschen-artigen Filters/Dialysators strömungstechnisch mittels gehäuseintegrierter (Gehäusewandintegrierter) Dialysatorkupplungen/Anschluss-Kupplungen der Maschine zur extrakorporalen Blutreinigung zu verbinden, insbesondere unmittelbar (ohne externe Verbindungsschläuche) zu verbinden, sowie eine derart dimensionierte Filteraufnahme bereitzustellen, dass die Verwendung ungeeigneter Filtermodule verhindert wird.

Nach der Erfindung sind die Anschluss-Kupplungen, mit denen der Filter/Dialysator an die internen Dialysierflüssigkeitsleitungen der Maschine angeschlossen wird, in deren Maschinengehäuse integriert bzw. sitzen unmittelbar an der Außenseite einer Gehäusewand der Maschine. Das bedeutet, dass die Anschluss-Kupplungen in einer für einen Nutzer der Maschine gut zugänglichen Weise mittelbar oder unmittelbar angeordnet, festgelegt oder ausgebildet sein können.

Die Anschluss-Kupplungen an der Maschine sind vorzugsweise entsprechend der Geometrie und relativen Lage der Dialysierflüssigkeitsanschlüsse des Dialysators zueinander positioniert, derart, dass beide Dialysierflüssigkeitsanschlüsse des Dialysators jeweils gleichzeitig mit der entsprechenden Anschluss-Kupplung der Maschine verbunden, vorzugsweise in diese eingeschoben, aufgesteckt oder aufgeschraubt werden können. Sobald die beiden Dialysierflüssigkeitsanschlüsse des Dialysators in bestimmungsgemäßer Weise in der jeweiligen Anschluss-Kupplung auf Seiten der Maschine sitzen, ist der Dialysierflüssigkeitskreislauf der Maschine geschlossen. Vorzugweise sind die Dialysierflüssigkeitsanschlüsse des Dialysators dann mit einer zum Halten des gesamten Dialysators an der Maschine ausreichenden Kraft gehalten. D.h. die maschinenseitigen Anschluss-Kupplungen sind optional zusätzlich so gestaltet, dass sie für das mechanische Halten des Filters/Dialysators am Maschinengehäuse im normalen Filterbetrieb ausreichend sind und daher die aus dem Stand der Technik bekannten Halteklammern oder Arme ersetzen.

Nach einer Ausführungsform der Erfindung ist der Dialysator in der Aufnahmemulde durch Reibschluss, Formschluss oder Kraftschluss gehalten. Alternativ oder zusätzlich kann das Gehäuse eine Klemmeinrichtung zum klemmenden Halten des Dialysators aufweisen.

Außerdem kann es vorgesehen sein, die maschinenseitigen und/oder dialysatorseitigen Anschlüsse nicht fix an der Gehäusewand bzw. Dialysatorkartusche anzuordnen, sondern mit einem Verstellmechanismus oder Adapter zu versehen, um deren Abstände zueinander wahlweise zu verändern. Eine Ausführungsform der Erfindung ist demnach dadurch gekennzeichnet, dass insbesondere die in das Gehäuse integrierten Anschluss-Kupplungen zueinander relativpositionierbar sind. Durch derart zueinander relativpositionierbare Kupplungen kann die Dialysemaschine flexibel mit Dialysatoren unterschiedlicher Größe oder Ausgestaltung genutzt werden. Vorzugsweise sind die Anschluss-Kupplungen zueinander relativpositionierbar und in der entsprechenden Relativposition lagefixierbar. Dazu kann zumindest eine der Anschluss-Kupplungen zum Beispiel mit einem Klemm- oder Schraubmechanismus versehen sein. Bei losem Mechanismus ist dann die zumindest eine Anschluss-Kupplung relativ zum Gehäuse positionierbar. Durch Sperren des Mechanismus ist die zumindest eine Anschluss-Kupplung in der gewünschten Weise zum Gehäuse klemm-/ arretierbar. Eine besonders einfache und nutzerfreundliche Positionierbarkeit der Anschluss-Kupplungen kann bewirkt werden, indem zumindest eine der Anschluss-Kupplungen im Gehäuse in einer Führung, zum Beispiel in einem Langloch, Schlitz oder Nut, verschiebbar aufgenommen ist.

Nach einer Ausführungsform der Erfindung sind die in das Gehäuse integrierten Anschluss-Kupplungen Schnellkupplungen zum Einschieben von Dialysierflüssigkeitsanschlüssen des Dialysators. Es ist besonders vorteilhaft, wenn diese auf den jeweiligen Dialysierflüssigkeitsanschluss des Dialysators eine zum Festlegen des Dialysators am Gehäuse geeignete, vorbestimmte und definierte Haltekraft ausüben. Dieses kann mittels Reibschluss und/oder Formschluss und/oder Kraftschluss erfolgen, zum Beispiel mittels einer Klemmung, eines Riegelmechanismus oder eines Rastmechanismus, die bei bestimmungsgemäßer Anordnung des Dialysators an der Maschine zur Wirkung kommen.

Nach einer weiteren Ausführungsform weist die Dialysemaschine des Weiteren ein (einziges/gemeinsames) Deckelelement zum Verschließen, insbesondere zum hermetisch dichten Verschließen, des Klemm-Anschlusses der Zuleitung (Frischdialysierflüssigkeitszuleitung der Dialysemaschine) und (gleichzeitig) des Anschlusses der Ableitung (Brauchdialysierflüssigkeitsableitung der Dialysemaschine) auf. Das (einzige) Deckelelement kann insbesondere einen Fluidkanal aufweisen, über den der Anschluss der Zuleitung mit dem Anschluss der Ableitung verbunden und kurzgeschlossen ist, wenn das Deckelelement die Anschlüsse verschließend am Gehäuse der Blutbehandlungsmaschine angeordnet ist. Ein solches Deckelelement kann besonders einfach als Desinfektionsklappe bei Reinigungs- und Sterilisierungsvorgängen der Internfluidik der Dialysemaschine genutzt werden.

Eine besonders nutzerfreundliche Ausführungsform kann vorsehen, dass das Deckelelement mittels eines Gelenks oder eines Scharniers bewegbar am maschinenseitigen Gehäuse gehalten ist, so dass es während eines Einsatzes der Maschine (Dialyse) mit an den Anschluss-Kupplungen gehaltenem Dialysator nicht verloren gehen kann.

Mit der Dialysemaschine nach der Erfindung können in vorteilhafter Weise zahlreiche verschiedene Therapieformen, wie zum Beispiel Hämodialyse (HD), Hämofiltration (HF) oder Hämodiafiltration (HDF) durchgeführt werden.

Abschließend lässt sich die vorliegende Erfindung zusammenfassend so beschreiben, die Dialysierflüssigkeitsanschlüsse eines Dialysators bzw. an einem Dialysator strömungstechnisch mittels gehäuseintegrierter Dialysator-Anschlusskupplungen einer Maschine zur extrakorporalen Blutreinigung direkt, bzw. ohne Verwendung von externen Verbindungschläuchen zu verbinden. In der Dialysemaschine (-gerät) können die Anschluss-Kupplungen zum Anschluss des Dialysators im Gehäuse integriert ausgebildet sein. Das Gehäuse kann hierfür optional eine Mulde zur räumlichen (Teil-)Aufnahme des Dialysators ausbilden oder eine ebene Fläche darstellen. Zur Desinfektion können die beiden Anschluss-Kupplungen für die Zu-/Abfuhr von Dialysierflüssigikeit zum/vom Dialysator über ein Verbindungselement (Anschlussbrücke) verbunden werden, das vorzugsweise von außerhalb der Maschine einfach auf die beiden Anschluss-Kupplungen aufgesetzt werden kann. Somit ist die Desinfektion der Anschlusskomponenten gewährleistet.

Wird der Dialysator angeschlossen, ist das Verbindungselement zu entfernen. Dies kann hierfür über ein Gelenk an dem Gehäuse beweglich angelenkt sein. Die Anschluss-Kupplungen sind so ausgeführt, dass eine leichte Verrastung des Dialysators in/an den Anschluss-Kupplungen möglich ist. Um verschiedene Dialysatorgrößen verwenden zu können, kann der Abstand der Anschluss-Kupplungen mit Hilfe eines Verstellmechanismus oder Adapters variabel verändert werden. Zur Entlastung der Anschlussstellen und zur Fixierung des Dialysators kann ein gabel- oder klemmenförmiges Führungselement zur Gewichtskraftaufnahme des Dialysators in den Aufbau integriert sein. Zum Entfernen des Dialysators kann eine zentrale Entkupplungsvorrichtung bzw. Auswerffunktion integriert sein.

Durch die Erfindung können insbesondere die folgenden Vorteile bewirkt werden:
- Es führen keine freiliegenden Schläuche vom Gerät zum Dialysator.
- Infolgedessen ist der Temperaturverlust der Dialysierflüssigkeit zwischen dem Dialysator und den endseitigen Kupplungen der Dialysierflüssigkeitsleitungen gering oder sogar nicht vorhanden, anders als bei in bekannter Weise durch Schläuche an die Dialysemaschine angeschlossenen Dialysatoren.
- Der Dialysatorbereich ist deutlich aufgeräumter, insbesondere kann es nicht zu einem Verwirren von Dialysierflüssigkeitsschläuchen mit den Blutschläuchen kommen.
- Es können keine Schläuche an oder durch nebenstehende Betten abgerissen/abgeklemmt werden.
- Die Anwenderfreundlichkeit ist durch die mittels der Erfindung bewirkte klare, aufgeräumte Anordnung und Optik gesteigert.
- In besonders vorteilhafter Weise ist eine Einhandbedienung, also ein Anschließen und/oder Abnehmen eines Dialysators an der Dialysemaschine möglich.
- Rüstzeiten sind gegenüber dem Stand der Technik deutlich verkürzt.

Die Erfindung wird im Folgenden anhand beispielhafter, nicht einschränkender und in den angehängten Figuren gezeigter Ausführungsformen näher erläutert. Dabei zeigt:
Figur 1 eine schematische Aufsicht auf einen Anschlussbereich für einen Dialysator an einem Gehäuse einer Dialysemaschine nach der Erfindung mit geschlossener Desinfektionsklappe,
Figur 2 eine schematische Aufsicht gemäß Figur 1 mit geöffneter Desinfektionsklappe und
Figur 3 eine schematische Aufsicht gemäß Figur 2 mit einem mit der Internfluidik der Dialysemaschine verbundenen und am Gehäuse gehaltenen Dialysator.

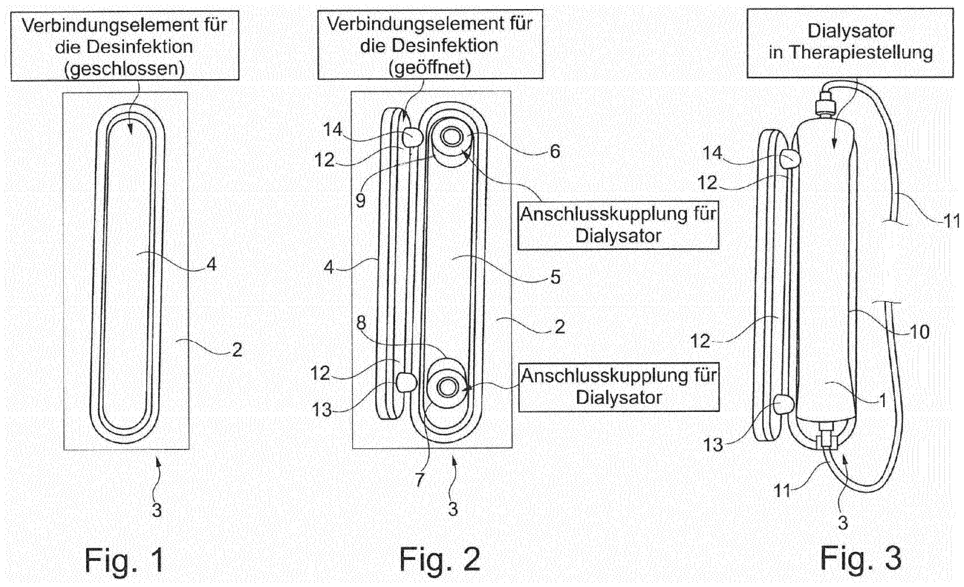

Die Figuren 1 und 2 zeigen jeweils einen Anschlussbereich für einen Dialysator 1 an einem Gehäuse 2 einer Dialysemaschine 3. Dabei verdeutlicht Figur 2 die Erfindung mit offener Desinfektionsklappe (abgenommener Verbindungsbrücke) 4 und Figur 1 die Erfindung mit geschlossener Desinfektionsklappe (aufgesetzter Verbindungsbrücke) 4. In das Gehäuse 2 der Dialysemaschine 3 ist eine Ausnehmung in Form einer Vertiefung/ Mulde 5 eingebracht. Die Vertiefung 5 ist von außen betrachtet konkav, das heißt geht in die Zeichnungsebenen der Figuren 1 und 2 hinein.

In der Vertiefung 5 sind eine erste Anschluss-Kupplung 6 (nachfolgend einfach als erste Kupplung bezeichnet) und eine zweite Anschluss-Kupplung 7 (nachfolgend einfach als zweite Kupplung bezeichnet) angeordnet. Sie sind in auf dem Boden der Vertiefung 5 ausgebildeten und in den Figuren etwas erkennbaren Langlöchern 8, 9 eingebracht und können durch Verschrauben mit diesen verklemmt werden. Infolge ihrer Führung in den Langlöchern 8, 9 sind die beiden Kupplungen 6, 7 zueinander in Richtung A relativpositionierbar. Die erste Kupplung 5 ist strömungstechnisch mit einer in den Figuren nicht dargestellten Frischdialysierflüssigkeitszuleitung verbunden, während die zweite Kupplung 6 mit einer in den Figuren ebenfalls nicht dargestellten Brauchdialysierflüssigkeitsableitung verbunden ist.

Figur 2 zeigt den Anschlussbereich bei geöffneter Desinfektionsklappe 4. In diesem Zustand wird der Dialysator 1, wie in Figur 3 dargestellt ist, in den Anschlussbereich eingesetzt. Dabei werden dessen an seiner Umfangsfläche 10 radial nach außen ragende Dialysierflüssigkeitsanschlüsse, die in den Figuren nicht erkennbar sind, in die jeweils entsprechende Kupplung 5, 6 eingesetzt oder eingeschoben. Bei Erreichen der bestimmungsgemäßen Endlage des Dialysators 1 am Gehäuse 2 sind die Dialysierflüssigkeitsseite des Dialysators 1, die Frischdialysierflüssigkeitszuleitung und die Brauchdialysierflüssigkeitsableitung strömungstechnisch miteinander verbunden. Des Weiteren ist der Dialysator 1 mit seinen Dialysierflüssigkeitsanschlüssen in den Kupplungen 5, 6 gehalten und am Gehäuse 2 abnehmbar lagefixiert. Bis auf Schläuche 11 des extrakorporalen Blutkreislaufs sind keine Schläuche im Anschlussbereich vorhanden, so dass dieser sehr übersichtlich und aufgeräumt ist.

Die Desinfektionsklappe 4 ist mittels eines Gelenks 12 (Scharnier) am Gehäuse 2 beweglich festgelegt. An ihrer im geschlossenen Zustand dem Gehäuse 2 zugewandten Seite ist sie mit Verschlussstopfen 13, 14 versehen, die zum Eingreifen in und Abdichten der Kupplungen 5, 6 ausgebildet und bestimmt sind. Die Verschlussstopfen 13, 14 können nach einer Form der Erfindung als Hohlzapfen ausgebildet sein und mit einem in den Figuren nicht dargestellten und in der Desinfektionsklappe ausgebildeten Verbindungskanal miteinander verbunden sein. Bei einer solchen Ausbildung können die beiden Kupplungen 5, 6 durch Schließen der Desinfektionsklappe 4 kurzgeschlossen werden, zum Beispiel zu Spülungs- und Reinigungszwecken.

### Bezugszeichen

1 Dialysator
2 Gehäuse
3 Dialysemaschine
4 Desinfektionsklappe
5 Vertiefung
6 Kupplung
7 Kupplung
8 Langloch
9 Langloch
10 Umfangsfläche
11 Schlauch extrakorporaler Blutkreislauf
12 Gelenk, Scharnier
13 Verschlussstopfen
14 Verschlussstopfen

## Patentansprüche

1. Extrakorporale Blutbehandlungsmaschine, vorzugsweise Dialysemaschine (3) mit einer maschineninternen Fluidik für eine Blutbehandlungsflüssigkeit und einem die Fluidik zumindest bereichsweise umgebenden Maschinengehäuse (2), wobei die Fluidik eine Zuleitung für frische Blutbehandlungsflüssigkeit zu einem Filter, vorzugsweise Dialysator (1) und eine Ableitung für verbrauchte Blutbehandlungsflüssigkeit vom Filter (1) aufweist, die jeweils endseitig mit einem Anschluss zur lösbaren Kopplung des Filters (1) an der maschineninternen Fluidik versehen sind, **wobei** der Anschluss der Zuleitung und der Anschluss der Ableitung jeweils als in das Maschinengehäuse (2) integrierte Anschluss-Kupplung (6, 7) ausgebildet sind und die Anschluss-Kupplungen (6, 7) in einer außenseitig im Maschinengehäuse (2) ausgebildeten oder angeordneten konkaven Ausnehmung (5) angeordnet sind, **dadurch gekennzeichnet, dass** die Ausnehmung (5) hinsichtlich ihrer Größe und Geometrie derart ausgebildet ist, dass sie eine Aufnahmemulde für den Filter (1) ausbildet, deren Abmessungen an den für die Blutbehandlungsmaschine (3) konkret vorgesehenen Filter (1) angepasst sind, derart, dass die Verwendung eines ungeeigneten Filters mit entsprechend unpassenden Abmessungen verhinderbar ist.

2. Extrakorporale Blutbehandlungsmaschine (3) nach Anspruch 1, **dadurch gekennzeichnet, dass** der Filter (1) in der Aufnahmemulde durch einen mit der Aufnahmemulde eingehenden Reibschluss, Formschluss oder Kraftschluss gehalten ist.

3. Extrakorporale Blutbehandlungsmaschine (3) nach einem der vorstehenden Ansprüche, **dadurch gekennzeichnet, dass** das Maschinengehäuse (2) eine Klemmeinrichtung zum klemmenden Halten des Filters (1) aufweist.

4. Extrakorporale Blutbehandlungsmaschine (3) nach einem der vorstehenden Ansprüche, **dadurch gekennzeichnet, dass** die in das Maschinengehäuse (2) integrierten Anschluss-Kupplungen (6, 7) zueinander relativpositionierbar sind, insbesondere zumindest eine der Kupplungen (6, 7) im Maschinengehäuse (2) in einer Führung (8, 9) verschiebbar aufgenommen ist.

5. Extrakorporale Blutbehandlungsmaschine (3) nach einem der vorstehenden Ansprüche, **dadurch gekennzeichnet, dass** die in das Maschinengehäuse (2) integrierten Anschluss-Kupplungen (6, 7) Schnellkupplungen zum Einschieben von Dialysierflüssigkeitsanschlüssen des Filters (1) sind, die auf den jeweiligen Dialysierflüssigkeitsanschluss eine zum Festlegen des Filters (1) am Maschinengehäuse (2) geeignete, vorbestimmte und definierte Haltekraft ausüben.

6. Extrakorporale Blutbehandlungsmaschine (3) nach einem der vorstehenden Ansprüche, **dadurch gekennzeichnet, dass** diese des Weiteren ein Deckelelement (4) zum Verschließen der Anschluss-Kupplung der Zuleitung und der Anschluss-Kupplung der Ableitung aufweist.

7. Extrakorporale Blutbehandlungsmaschine (3) nach Anspruch 6, **dadurch gekennzeichnet, dass** das Deckelelement (4) einen Fluidkanal aufweist, über den die Anschluss-Kupplung der Zuleitung (6) mit der Anschluss-Kupplung der Ableitung (7) verbunden und kurzgeschlossen ist, wenn das Deckelelement (4) die Anschluss-Kupplungen verschließend am Maschinengehäuse (2) angeordnet ist.

8. Extrakorporale Blutbehandlungsmaschine (3) nach Anspruch 6 oder 7,
**dadurch gekennzeichnet, dass** das Deckelelement (4) mittels eines Gelenks 12) oder eines Scharniers bewegbar am Maschinengehäuse (2) gehalten ist.

## Claims

1. Extracorporeal blood treatment machine, preferably dialysis machine (3), with machine-internal fluidics for a blood treatment fluid and a machine housing (2) at least in sections surrounding the fluidics, wherein the fluidics comprise an input line for fresh blood treatment fluid to a filter, preferably a dialyser (1), and an output line for used blood treatment fluid from the filter (1), that are each equipped at their end side with a connection port for releasably coupling the filter (1) to the machine-internal fluidics, **wherein** the connection port of the input line and the connection port of the output line each are formed as a connection coupling (6, 7) integrated into the machine housing (2), and the connection couplings (6, 7) are arranged in a concave recess (5) formed or arranged at an outer side in the machine housing (2), **characterised in that** the recess (5) is configured with regard to its size and geometry such that it forms a reception trough for the filter (1), the dimensioning of which is adapted to that exact filter specifically intended for the blood treatment machine, such that using an unsuitable filter is preventable with corresponding unsuitable dimensions.

2. Extracorporeal blood treatment machine (3) according to claim 3, **characterised in that** the filter (1) is held in the reception trough by friction-fit, form-fit or force-fit established with the reception trough.

3. Extracorporeal blood treatment machine (3) according to one of the preceding claims, **characterised in that** the machine housing (2) comprises a clamping assembly for holding the filter (1) by clamping.

4. Extracorporeal blood treatment machine (3) according to one of the preceding claims, **characterised in that** the connection couplings (6, 7) integrated into the machine housing (2) can be positioned relative to each other, in particular, at least one of the couplings (6, 7) is slidably received in the machine housing (2) in a guide (8, 9).

5. Extracorporeal blood treatment machine (3) according to one of the preceding claims, **characterised in that** the connection couplings (6, 7) integrated into the machine housing (2) are quick couplings for insertion of dialysis fluid ports of the filter (1), that exert on the respective dialysis fluid port a predetermined and defined holding force that is suitable for affixing the filter (1) at the machine housing (2).

6. Extracorporeal blood treatment machine (3) according to one of the preceding claims, **characterised in that** the extracorporeal blood treatment machine further comprises a lid element (4) for closing the connection coupling of the input line and the connection coupling of the output line.

7. Extracorporeal blood treatment machine (3) according to claim 6, **characterised in that** the lid element (4) comprises a fluid passage, via which the connection coupling of the input line (6) is connected and short-circuited with the connection coupling of the output line (7), when the lid element (4) is arranged at the machine housing (2) in a manner to close the connection couplings.

8. Extracorporeal blood treatment machine (3) according to claim 6 or 7, **characterised in that** the lid element (4) is movably held at the machine housing (2) by a joint (12) or a hinge.

## Revendications

1. Machine de traitement du sang extracorporelle, de préférence machine de dialyse (3) comprenant une fluidique interne à la machine pour un fluide de traitement du sang et un carter de machine (2) entourant au moins partiellement la fluidique, dans laquelle la fluidique présente une conduite d'alimentation pour alimenter un fluide de traitement du sang frais vers un filtre, de préférence un dialyseur (1) et une conduite d'évacuation pour évacuer le fluide de traitement du sang usé du filtre (1), qui sont prévues respectivement côté extrémité d'un raccordement pour le couplage amovible du filtre (1) à la fluidique interne à la machine, **dans laquelle** le raccordement de la conduite d'alimentation et le raccordement de la conduite d'évacuation sont conçus respectivement en tant que couplage de raccordement (6, 7) intégré au carter de machine (2) et les couplages de raccordement (6, 7) sont disposés dans un évidement (5) concave conçu ou disposé d'un côté extérieur dans le carter de machine (2), **caractérisée en ce que** l'évidement (5) est conçu en ce qui concerne sa taille et sa géométrie de telle manière qu'il forme un creux d'évidement pour le filtre (1), dont les dimensions sont adaptées au filtre (1) prévu en particulier pour la machine de traitement du sang (3), de telle manière que l'utilisation d'un filtre inadéquat avec des dimensions inappropriées correspondantes peut être évitée.

2. Machine de traitement du sang extracorporelle (3) selon la revendication 1, **caractérisée en ce que** le filtre (1) est retenu dans le creux d'évidement par un frottement, un engagement positif ou une liaison de force en profondeur avec le creux d'évidement.

3. Machine de traitement du sang extracorporelle (3) selon l'une quelconque des revendications précédentes, **caractérisée en ce que** le carter de machine (2) présente un dispositif de serrage pour retenir par serrage le filtre (1).

4. Machine de traitement du sang extracorporelle (3) selon l'une quelconque des revendications précédentes, **caractérisée en ce que** les couplages de raccordement (6, 7) intégrés au carter de machine (2) peuvent être positionnés de manière relative les uns par rapport aux autres, en particulier au moins l'un des couplages (6, 7) dans le carter de machine (2) est reçu de manière à pouvoir coulisser dans une glissière (8, 9).

5. Machine de traitement du sang extracorporelle (3) selon l'une quelconque des revendications précédentes, **caractérisée en ce que** les couplages de raccordement (6, 7) intégrés au carter de machine (2) sont des couplages rapides pour insérer des raccordements de fluide de dialyse du filtre (1), qui exercent sur le raccordement de fluide de dialyse respectif une force de retenue adéquate, prédéterminée et définie pour fixer le filtre (1) au niveau du carter de machine (2).

6. Machine de traitement du sang extracorporelle (3) selon l'une quelconque des revendications précédentes, **caractérisée en ce que** celle-ci présente en outre un élément de couvercle (4) pour verrouiller le couplage de raccordement de la conduite d'alimentation et le couplage de raccordement de la conduite d'évacuation.

7. Machine de traitement du sang extracorporelle (3) selon la revendication 6, **caractérisée en ce que** l'élément de couvercle (4) présente un canal de fluide, sur lequel le couplage de raccordement de la conduite d'alimentation (6) est relié et court-circuité avec le couplage de raccordement de la conduite d'évacuation (7), lorsque l'élément de couvercle (4) est disposé au niveau du carter de la machine (2) de manière à verrouiller les couplages de raccordement.

8. Machine de traitement du sang extracorporelle (3) selon la revendication 6 ou 7, **caractérisée en ce que** l'élément de couvercle (4) est retenu au moyen d'une articulation (12) ou d'une charnière de manière mobile au niveau du carter de machine (2).
